# EUROPÄISCHE PATENTSCHRIFT

(11) **EP 1 960 023 B1**
(45) Veröffentlichungstag und Bekanntmachung des Hinweises auf die Patenterteilung: **26.02.2014**
(21) Anmeldenummer: 06827955.3
(22) Anmeldetag: 18.12.2006
(51) Int. Cl.: A61M 16/00, A61M 16/04

(54) **JET-ENDOSKOP**
JET-ENDOSCOPE
ENDOSCOPE A JET

(30) Priorität: 16.12.2005 AT 20182005
(43) Veröffentlichungstag der Anmeldung: 27.08.2008
(73) Patentinhaber: Carl Reiner GmbH, 1090 Wien (AT)
(72) Erfinder: LIRSCH, Dominik, A-2460 Bruckneudorf (AT); KÖLBL, Robert, A-3430 Tulln (AT)
(74) Vertreter: Keschmann, Marc
(86) Internationale Anmeldenummer: PCT/AT2006/000522
(87) Internationale Veröffentlichungsnummer: WO 2007/068023

(56) Entgegenhaltungen:
- WO-A-96/34643
- WO-A-96/39216
- DE-A1- 19 963 740
- GB-A- 2 033 759
- US-A- 4 644 947
- US-A- 4 821 709

## Beschreibung

Die Erfindung bezieht sich auf ein Jet-Endoskop für die Beatmung und Eingriffe im operativen Anwendungsbereich mit Anschlüssen für Gasleitungen, an welche verschiedene Beatmungsgase mit unterschiedlicher Pulsationsfrequenz anschließbar sind, wobei Anschlüsse für wenigstens zwei Kanäle für die Einleitung von Gasen mit unterschiedlicher Pulsationsfrequenz und für eine Gasanalyse oder eine Druckmessung vorgesehen sind.

Jet-Endoskope der eingangs genannten Art umfassen in aller Regel einen Tubus, an welchen Anschlüsse für Beatmungsgase oder Sauerstoff- bzw. CO₂-Messeinrichtungen angeschlossen werden können. Bekannte Einrichtung der eingangs genannten Art haben neben einer Hauptachse an ihrem distalen Ende in einen T-Balken mündende Anschlüsse, welche im Inneren des Tubus, beispielsweise eines Laryngoskops enden. Die US 5, 752, 506 A sowie die US 5,165,398 A zeigen hierbei Beatmungseinrichtungen mit überlagerter Hochfrequenzbeatmung, wobei beispielsweise über einen speziellen Adapter auch ein Atemwegsdruck abgenommen werden kann.

Bei der GB 2063686 A sowie der DE 2947659 A1 wird ein Hoch-frequenzbeatmungssystem beschrieben, in welchem eine Messleitung zur Aufnahme von Atemgasproben vorgesehen ist. Die DE 2847681 A beschreibt beispielsweise eine Trachealkanüle, in deren distalem Ende zwei oder drei Düsen enden, wobei eine dieser Düsen einer Messleitung zugeordnet ist.

Die DE 19963740 A1 beschreibt einen Adapter zur Verbindung eines Jet-Beatmungsgerätes mit einem Endotrachealtubus. Der Adapter weist einen rohrförmigen Abschnitt mit einer an den Tubus anschließbaren Öffnung und mit einer Öffnung zum Ansaugen von Beatmungsgas mittels des Venturieffektes sowie Zufuhrrohre für den Jet-Gasstrom auf.

Die US 4821709 A beschreibt ein Verfahren zur Hochfrequenzventilation.

Die Erfindung zielt nun darauf ab, ein Jet-Endoskop der eingangs genannten Art zu schaffen, mit welchem neben der intraoperativen Hochfrequenzbeatmung eines Patienten während eines invasiven Eingriffs im operativen Anwendungsbereich eine Überwachung der relevanten Atemgasparameter ermöglicht wird, welche die laufende Anpassung der jeweils erforderlichen Beatmungsmaßnahmen wesentlich erleichtert und unverfälschte Messungen ermöglicht.

Zur Lösung dieser Aufgabe ist das erfindungsgemäße Jet-Endoskop im wesentlichen dadurch gekennzeichnet, dass wenigstens vier Anschlüsse für wenigstens zwei in Achsrichtung des Jet-Endoskops versetzt angeordnete und unter spitzem Winkel mit der Achse in Düsen mündende Kanäle für die Einleitung von Gasen mit unterschiedlicher Pulsationsfrequenz und wenigstens zwei weitere gegenüber den Beatmungskanälen distale Kanäle für eine gesonderte Gasanalyse und eine gesonderte Druckmessung angeordnet sind. Durch die in axialer Richtung versetzte Mündung der Beatmungskanäle für die Zufuhr von Beatmungsgasen mit unterschiedlichen Pulsationsfrequenzen wird zunächst eine optimale Überlagerung von hoch- und normofrequenter Beatmung ermöglicht, wobei gleichzeitig ein entsprechend großer lichter Querschnitt für das Einführen von medizinischen Instrumenten für invasive Applikationen freigehalten wird. Dadurch, dass nun zusätzlich wenigstens zwei weitere gegenüber den Beatmungskanälen distale Kanäle für eine gesonderte Gasanalyse und eine gesonderte Druckmessung angeordnet sind, gelingt es, die Gasanalyse unabhängig von Verfälschungen durch Druckmessungen vorzunehmen und umgekehrt Druckmessungen ohne Verfälschung durch die Entnahme von Gasen für die Gasanalyse vorzunehmen. In beiden Fällen lassen sich somit permanent am distalen Ende der Atemwegdruck sowie die Sauerstoff- bzw. CO₂-Situation überwachen und die Beatmungsparameter, wie sie über die beiden dem proximalen Ende näher liegenden Düsen eingestellt werden, laufend an die Erfordernisse anpassen. Die Trennung des Kanals für die Abnahme des Atemwegdrucks von der Sonde für die Entnahme von Gasen für die Sauerstoff- und/oder CO₂-Messung führt hierbei zu der gewünschten Verbesserung der korrekten.Überwachung der Atemsituation und erlaubt eine kontinuierliche Regelung der relevanten Atemparameter.

Mit Vorteil ist die erfindungsgemäße Ausbildung hierbei so getroffen, dass der Kanal für die Druckmessung in Abstand von dem Kanal für die Gasanalysemessung und axial zwischen den Beatmungskanälen und dem Kanal für die Gasanalyse mündet. Eine derartige Anordnung des Kanals für die Druckmessung führt zu besonders exakten Messwerten, welche zur Regelung der Beatmungsparameter herangezogen werden kann.

Prinzipiell wird bei derartigen Jet-Endoskopen das proximale Ende offen gehalten, sodass gemeinsam mit den normofrequent bzw. hochfrequent eingestoßenen Beatmungsgasen eine Ansaugung von Umgebungsluft erfolgen kann. Der entsprechende Kanal muss am proximalen Ende des Jet-Endoskops einen entsprechend großen Querschnitt aufweisen, um das ungehinderte Einbringen von Operationsinstrumenten zu ermöglichen. Erfindungsgemäß wird nun mit Vorteil die Ausbildung so weitergebildet, dass wenigstens ein weiterer Anschluss für eine Atemgaskonditionierung am proximalen offenen Ende des Jet-Endoskops vorgesehen ist, welcher spitzwinkelig zur Achse des Jet-Endoskops mündet. Ein derartiger spitzwinkelig zur Achse des Jet-Endoskops mündender zusätzlicher Kanal erlaubt die Zufuhr von befeuchteter Luft oder anderweitig konditionierter Luft, ohne den lichten Querschnitt für das Einbringen von Operationsinstrumenten zu beeinträchtigen.

Eine besonders geringe Beeinträchtigung des lichten Querschnitts bei gleichzeitig entsprechend hoher mechanischer Stabilität lässt sich dadurch verwirklichen, dass die für die Anschlüsse vorgesehenen Kanäle als an der Außenwand des Tubus verlaufende Leitungen in im wesentlichen axialer Richtung des Jet-Endoskops ausgebildet sind, deren distale Enden in jeweils voneinander verschiedenen Radialebenen münden.

Um eine völlig unbeeinträchtigte und fehlerfreie Messung und Gasanalyse bei gleichzeitig mit unterschiedlicher Pulsationsfrequenz vorgenommener Beatmung vornehmen zu können, ist erfindungsgemäß die Ausbildung bevorzugt so gestaltet, däss der axiale Abstand zwischen den Mündungen der Atemgase und den Mündungen der Kanäle für die Gasanalyse und die Druckmessung wenigstens 40 mm, vorzugsweise wenigstens 50 mm beträgt. Um gleichzeitig eine Gasanalyse und eine fehlerfreie Druckmessung zu ermöglichen, ist mit Vorteil die Ausbildung so getroffen, dass die in axialer Richtung um wenigstens 5 mm versetzt angeordneten Mündungen der Kanäle für die Gasanalyse und die gesonderte Druckmessung auch in Umfangsrichtung des Tubusmantels versetzt angeordnet sind. Bei dieser Ausbildung ist zu berücksichtigen, dass für die Zwecke der Gasanalyse und insbesondere der Bestimmung von O₂ und CO₂ Gasmengen in der Größenordnung von etwa 200 ml/min abgezogen werden. An der in Umfangsrichtung und in axialer Richtung versetzten Mündung des Anschlusses' für die. Druckmessung kann der Atemwegdruck dann besonders fehlerfrei ermittelt werden, wenn diese Mündung gleichzeitig mit einer geringeren Menge an Atemgas gespült wird. Im dem distalen Ende näheren Bereich der Mündung der Leitungen für die *Atemgase entsteht* bei wesentlich größeren *eingestoßenen* Gasmengen ein entsprechender Unterdruck, der eine Atemwegdruckmessung wiederum verfälschen würde. Die erfindungsgemäß vorgesehenen Mindestabstände erlauben die Entkopplung der einzelnen Einflussfaktoren, welche eine Druckmessung verzerren würden.

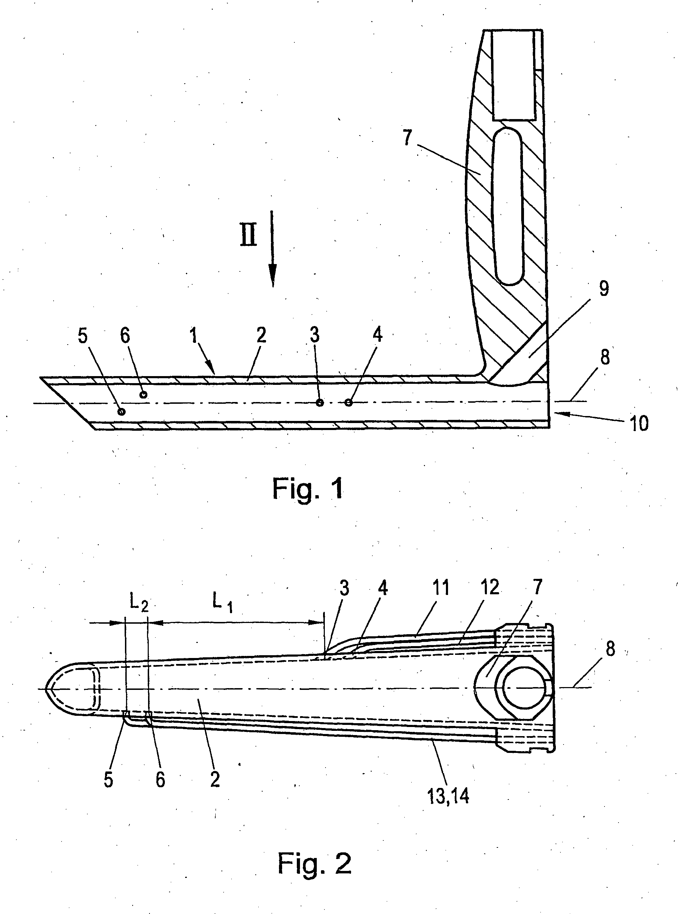

Die Erfindung wird nachfolgend anhand eines in der Zeichnung schematisch dargestellten Ausführungsbeispiels des erfindungsgemäßen Jet-Endoskops näher erläutert. In dieser zeigen Fig.1 einen Axialschnitt durch das Jet-Endoskop und Fig.2 eine Ansicht in Richtung des Pfeils II der Fig.1 mit den Details für die Mündungen der einzelnen Leitungen.

In Fig.1 ist mit 1 ein Jet-Endoskop bezeichnet, dessen Tubus 2 schematisch mit 3 und 4 bezeichnete Mündungen für Atemgase und mit 5 und 6 bezeichnete Mündungen für eine Atemwegsdruckmessung und eine Gasanalyse aufweist. Es ist weiters ein Griffstück 7 ersichtlich, wobei spitzwinkelig zur Richtung der Achse 8 des Tubus ein weiterer Anschluss 9 für die Zufuhr von konditionierter und befeuchteter Atemluft vorgesehen ist. Diese befeuchtete Atemluft kann zusätzlich zu der über das proximale offene Ende 10 angesaugten Luft zur Beatmung herangezogen werden. Das proximale offene Ende 10 dient hierbei auch dem Einführen von Operationsinstrumenten. Bei der Darstellung nach Fig.1 wird über die mit 4 angedeutete Position der Mündung niederfrequentes Jetgas eingestoßen, an der mit 3 bezeichneten Position erfolgt der Einstoß von hochfrequentem Jetgas, wobei sich im Inneren des Tubus 2 in der Nähe der als Jetdüsen ausgebildeten Mündung entsprechende Saugdruckverhältnisse einstellen.

Die entsprechenden Leitungen, welche zu den mit 3 und 4 angedeuteten Mündungspositionen führen, sind in Fig.2 mit 11 und 12 bezeichnet.

In Fig.2 sind darüber hinaus die Leitungen 13 und 14 zu einem Gasanalysemessgerät und einem Druckmessgerät eingezeichnet. Während die Kanäle 11 und 12 in der Zeichenebene der Fig.2 nebeneinander angeordnet sein können, sind die Kanäle 13 und 14 übereinander angeordnet, da die Mündungspositionen 5 und 6 dieser Kanäle in Umfangsrichtung versetzt angeordnet sind, wie dies in Fig.1 schematisch angedeutet ist. Der Abstand zwischen den Mündungen 5 und 6 beträgt mindestens 5 mm und ist mit L₂ bezeichnet. Der entsprechende Abstand L₁ zwischen der Mündung 6 und der in axialer Richtung zum proximalen Ende hin nächsten Mündung 3 beträgt mindestens 40 mm. Die für die Gasanalyse benötigte Gasmenge wird über die Mündung 5 näher dem distalen Ende des Tubus 2 angesaugt. Die in dem genannten Mindestabstand L₂ in Achsrichtung und in Umfangsrichtung versetzt angeordnete Mündung 6 dient hierbei der Messung des Atemwegdrucks.

## Patentansprüche

1. Jet-Endoskop für die Beatmung und Eingriffe im operativen Anwendungsbereich mit Anschlüssen für Gasleitungen, an welche verschiedene Beatmungsgase mit unterschiedlicher Pulsationsfrequenz anschließbar sind, wobei Anschlüsse für wenigstens zwei Kanäle für die Einleitung von Gasen mit unterschiedlicher Pulsationsfrequenz und für eine Gasanalyse oder eine Druckmessung vorgesehen sind, **dadurch gekennzeichnet, dass** wenigstens vier Anschlüsse für wenigstens zwei in Achsrichtung des Jet-Endoskops (1) versetzt und unter spitzem Winkel mit der Achse (8) mündende Kanäle (11,12) für die Einleitung von Gasen mit unterschiedlicher Pulsationsfrequenz und wenigstens zwei weitere gegenüber den Beatmungskanälen (11,12) distale Kanäle (1,14) für eine gesonderte Gasanalyse und eine gesonderte Druckmessung angeordnet sind.

2. Jet-Endoskop nach Anspruch 1, **dadurch gekennzeichnet, dass** der Kanal (13) für die Druckmessung in Abstand (L₂) von dem Kanal (14) für die Gasanalysemessung und axial zwischen den Beatmungskanälen (11,12) und dem Kanal (14) für die Gasanalyse mündet.

3. Jet-Endoskop nach Anspruch 1 oder 2, **dadurch gekennzeichnet, dass** wenigstens ein weiterer Anschluss (9) für eine Atemgaskonditionierung am proximalen offenen Ende des Jet-Endoskops (1) vorgesehen ist, welcher spitzwinkelig zur Achse (8) des Jet-Endoskops (1) mündet.

4. Jet-Endoskop nach Anspruch 1, 2 oder 3, **dadurch gekennzeichnet, dass** die am für die Anschlüsse vorgesehenen Kanäle (11,12,13,14) als an der Außenwand des Tubus verlaufende Kanäle in im wesentlichen axialer Richtung des Jet-Endoskops (1) ausgebildet sind, deren distale Enden in jeweils voneinander verschiedenen Radialebenen münden.

5. Jet-Endoskop nach einem der Ansprüche 1 bis 4, **dadurch gekennzeichnet, dass** der axiale Abstand zwischen den Mündungen (3,4) der Atemgase und den Mündungen (5,6) der Kanäle (13,14) für die Gasanalyse und die Druckmessung wenigstens 40 mm, vorzugsweise wenigstens 50 mm beträgt.

6. Jet-Endoskop nach einem der Anschlüsse 1 bis 5, **dadurch gekennzeichnet, dass** die in axialer Richtung um wenigstens 5 mm versetzt angeordneten Mündungen (5,6) der Kanäle (13,14) für die Gasanalyse und die gesonderte Druckmessung auch in Umfangsrichtung des Tubusmantels versetzt angeordnet sind.

## Claims

1. A jet endoscope for respiration and interventions in surgical applications, including connections for gas lines to which different respiratory gases with different pulsation frequencies can be connected, whereby connections for at least two channels for the injection of gases with different pulsation frequencies and for a gas analysis or a pressure measuring are provided, **characterized in that** at least four connections are provided for at least two channels (11,12) arranged in a relatively offset manner in the axial direction of the jet endoscope (1) while opening at acute angles with respect to said axis (8) for the injection of gases with different pulsation frequencies, and at least two further channels (13,14) provided distally to the respiratory channels (11,12) for a separate gas analysis and a separate pressure measurement, respectively.

2. A jet endoscope according to claim 1, **characterized in that** the channel (13) for pressure measurement opens at a distance (L₂) from the channel (14) for gas analysis measurement and axially between the respiratory channels (11,12) and the channel (14) for gas analysis.

3. A jet endoscope according to claim 1 or 2, **characterized in that** at least one further connection (9) for respiratory gas conditioning is provided on the proximal, open end of the jet endoscope (1), which further connection opens at an acute angle with respect to the axis (8) of the jet endoscope (1).

4. A jet endoscope according to claim 1, 2 or 3, **characterized in that** the channels (11,12,13,14) provided for the connections are configured as channels extending on the outer wall of the tube in the substantially axial direction of the jet endoscope (1) and whose distal ends each open in respectively different radial planes.

5. A jet endoscope according to any one of claims 1 to 4, **characterized in that** the axial distance between the ports (3,4) of the respiratory gases and the ports (5,6) of the channels (13,14) for gas analysis and pressure measurement is at least 40 mm and, preferably, at least 50 mm.

6. A jet endoscope according to any one of claims 1 to 5, **characterized in that** the ports (5, 6) of the channels (13,14) for gas analysis and separate pressure measurement, which are arranged to be offset in the axial direction by at least 5 mm, are also arranged to be offset in the peripheral direction of the tube jacket.

## Revendications

1. Endoscope à jet pour la respiration et des interventions dans le domaine d'utilisation opératoire, doté de raccords pour des conduites de gaz, auquel divers gaz respiratoires peuvent être raccordés avec une fréquence de pulsations différente, dans lequel des raccords sont prévus pour au moins deux canaux pour l'introduction de gaz avec une fréquence de pulsations différente et pour une analyse de gaz ou une mesure de pression, **caractérisé en ce qu'**au moins quatre raccords pour au moins deux canaux (11, 12) décalés en direction de l'axe de l'endoscope à jet (1) et débouchant à l'angle aigu avec l'axe (8) pour l'introduction de gaz avec une fréquence de pulsations différente et au moins deux autres canaux distaux (1, 14) sont disposés en face des canaux respiratoires (11, 12) pour une analyse de gaz distincte et une mesure de pression distincte.

2. Endoscope à jet selon la revendication 1, **caractérisé en ce que** le canal (13) pour la mesure de pression débouche à distance (L₂) du canal (14) pour la mesure de l'analyse de gaz, et axialement entre les canaux respiratoires (11, 12) et le canal (14) pour l'analyse de gaz.

3. Endoscope à jet selon la revendication 1 ou 2, **caractérisé en ce qu'**au moins un autre raccord (9) pour un conditionnement de gaz respiratoire est prévu à l'extrémité proximale ouverte de l'endoscope à jet (1) qui débouche à l'angle aigu par rapport à l'axe (8) de l'endoscope à jet (1).

4. Endoscope à jet selon la revendication 1, 2 ou 3, **caractérisé en ce que** les canaux prévus pour les raccords (11, 12, 13, 14) sont conçus comme des canaux évoluant sur la paroi extérieure du tube essentiellement en direction axiale de l'endoscope à jet (1), dont les extrémités distales débouchent sur des plans radiaux respectivement différents les uns des autres.

5. Endoscope à jet selon l'une des revendications 1 à 4, **caractérisé en ce que** la distance axiale entre les ouvertures (3, 4) des gaz respiratoires et les ouvertures (5, 6) des canaux (13, 14) pour l'analyse de gaz et la mesure de pression est d'au moins 40 mm, de préférence, d'au moins 50 mm.

6. Endoscope à jet selon l'une des revendications 1 à 5, **caractérisé en ce que**, en direction axiale, des ouvertures (5,6) des canaux (13, 14) décalées d'au moins 5 mm pour l'analyse de gaz et la mesure de pression distincte sont décalées également dans la direction périphérique de la paroi du tube.
